# EUROPEAN PATENT APPLICATION

(11) **EP 3 561 682 A1**
(43) Date of publication of application: **30.10.2019**
(21) Application number: 17885105.1
(22) Date of filing: 18.12.2017
(51) Int. Cl.: G06F 13/00, G06Q 50/00, G06Q 50/22, G16H 10/60

(54) **METHOD, PROGRAM, AND INFORMATION PROCESSING DEVICE**

(30) Priority: 21.12.2016 JP 2016248172; 09.06.2017 JP 2017114318
(71) Applicant: Finc Technologies Inc., Tokyo 1000006 (JP)
(72) Inventor: MIZOGUCHI, Yuji, Tokyo 100-0006 (JP); KONDO, Takao, Tokyo 100-0006 (JP)
(74) Representative: Klunker IP Patentanwälte PartG mbB
(86) International application number: PCT/JP2017/045250
(87) International publication number: WO 2018/117003

(57) **Abstract**

A predetermined user is supported behind the scenes with a variety of functions in order to promote messaging in a talk room in an instant messaging application. A method for messaging through a talk room is provided. This method includes the steps, executed by a virtual user implemented by a computer for assisting a first user, of dynamically generating a first message intended for the first user or a second user based on a history of messaging in the talk room and health information on the second user; providing the first message to the talk room in association with the first user; updating the history in response to the provision of the first message; and generating a first screen to be presented to the first user and a second screen to be presented to the second user, the first and second screens being associated with the talk room. The history is classified into a first group involved with the first user and the virtual user that perform the messaging, and a second group involved with the second user, and placed on each of the first screen and second screen over time.

## Description

### TECHNICAL FIELD

The present disclosure relates to a method, a program, and an information processing device for messaging through a talk room.

### BACKGROUND ART

An instant messaging (IM) service is known for enabling a plurality of users to interact with one another by performing messaging using terminals such as a smart phone and a tablet. A user can perform messaging through a dedicated virtual space ("talk room") by installing an application for IM service in his portable terminal, and linking accounts between the user and a partner user (becoming "friends").

The IM application provides a software robot user implemented by a software program, in addition to actual users who operate terminals. For example, Patent Document 1 discloses a software robot user which has an interpretation function. Assume, as an example, a Japanese user and a Korean user are in a friend relationship, and a software robot, provided as a "Korean translation user," is also added as a friend, such that the three parties participate in a talk room. When the Japanese user contributes a Japanese text message, the software robot, in response to the contributed message, immediately translates its contents into the Korean language, and contributes the translated message. Since the Korean user can understand the text message written by the Japanese user by viewing the translated message, a smooth communication can be made through the talk room. In this way, the software robot user is provided to perform limited behaviors, with respect to a predetermined function, that include participating in a talk room as an independent user having an account and making one response to one particular request.

### CITATION LIST

### PATENT DOCUMENT

[Patent Document 1] JP-A-2015-179519

### SUMMARY OF INVENTION

### TECHNICAL PROBLEM

It is an object of the present disclosure to provide messaging supporting technologies having a variety of functions, which behave to support a predetermined user behind the scenes, in order to promote messaging in a talk room, in an application for IM service. More particularly, it is an object of the present disclosure to provide messaging supporting technologies which allow part of behaviors of a predetermined user to be performed on behalf of the predetermined user.

### SOLUTION TO PROBLEM

According to one embodiment of the present disclosure, a method is provided for messaging through a talk room. This method includes the steps, executed by a virtual user implemented by a computer for assisting a first user, of dynamically generating a message intended for the first user or a second user based on a history of messaging in the talk room and health information on the second user; providing the first message to the talk room in association with the first user; updating the history in response to the provision of the first message; and generating a first screen to be presented to the first user and a second screen to be presented to the second user, where the first and second screens are associated with the talk room. The history is classified into a first group involved with the first user and the virtual user that perform the messaging, and a second group involved with the second user, and placed on each of the first screen and second screen over time.

Also, according to one embodiment of the disclosure, a method is provided for assisting a first user in messaging between the first user and a second user through a talk room. This method includes the steps, executed by a computer, of dynamically generating a message intended for the second user based on a history of messaging in the talk room and health information on the second user; acting on behalf of the first user by providing the message to the talk room in association with the first user; updating the history in response to the provision of the message; and generating a first screen to be presented to the first user and a second screen to be presented to the second user, where the first and second screens are associated with the talk room. The history is classified into a first group involved with the first user that perform the messaging and the computer, and a second group involved with the second user, and placed over time on each of the first screen and second screen.

Further, according to one embodiment of the disclosure, an information processing device for messaging through a talk room is provided. This information processing device comprises a management unit for managing a relationship between a first user that provides a service and a second user that is provided with the service, and managing a history of messaging between the first user and the second user in the talk room; a virtual user unit for implementing a virtual user for assisting the first user, including an analyzer unit for analyzing the history; a message generator unit for dynamically generating a message in accordance with the analysis and health information on the second user; a provision unit for providing the message to the talk room in association with the first user; and an update unit for updating the history with respect to the provided message; and a screen generator unit for generating a first screen to be presented to the first user and a second screen to be presented to the second user based on the updated history, where the first and second screens are associated with the talk room. The history is classified into a first group involved with the first user and the virtual user, that performed messaging and the second user, and placed on each of the first screen and second screen over time.

Each embodiment of the disclosure can implement a virtual user having a variety of functions for supporting a first user behind the scenes. The virtual user can autonomously determine a next operation in accordance with the progress of messaging. In this way, the messaging in the talk room can be promoted particularly by the virtual user performing messaging to the second user on behalf of the first user. In a scenario where an instructor makes advice for a student, communications between both parties can be made efficient and active.

### BRIEF DESCRIPTION OF DRAWINGS

Fig. 1 is a basic configuration diagram of an information processing system.
Fig. 2 is a hardware configuration diagram of an information processing device.
Fig. 3 is a hardware configuration diagram of a user terminal.
Fig. 4 is a functional block diagram of the information processing system.
Fig. 5 is an outline diagram showing the relationship between accounts in a talk room.
Fig. 6 is a screen image generated in a method according to one embodiment.
Fig. 7 is a processing flow diagram of the method according to the embodiment.
Fig. 8 is an outline diagram of tasks for a healthcare service program.
Fig. 9 is a screen image generated in a method according to one embodiment.
Fig. 10 is an outline diagram of a message setting information.
Fig. 11 is a tablar configuration diagram of a message history information.
Fig. 12A is a screen image generated in a method according to one embodiment.
Fig. 12B is a screen image generated in a method according to one embodiment.
Fig. 13 is a processing flow diagram of a method according to one embodiment.
Fig. 14 is a screen image generated in a method according to one embodiment.

### DESCRIPTION OF EMBODIMENTS

First, an illustrative embodiment of the disclosure will be described while listing components thereof. A computer-implemented method executed by a computer comprising a touch panel, according to an embodiment of the disclosure, may comprise the following configuration.
(Aspect 1) According to Embodiment 1, a method is provided for messaging through a talk room. This method includes the steps, executed by by a virtual user implemented by a computer for assisting the first user, of dynamically generating a message intended for a first user or a second user, based on the history of messaging in a talk room and health information on a second user; providing the message to the talk room in association with the first user; updating the history in response to the provision of the message; generating a first screen to be presented to the first user and a second screen to be presented to the second user, where the screens are associated with the talk room, wherein the history is classified into a first group involved with the first user and the virtual user that perform the messaging, and a second group involved with the second user, and placed on the first screen and second screen, respectively, over time. With the configuration as described, the virtual user having a variety of functions can be implemented to behave to support the first user behind the scenes, thus promoting the messaging in the talk room. The virtual user can autonomously determine a next operation in accordance with the progress of messaging. Also, the virtual user can be clearly demonstrated on a displayed screen, that it is a user for supporting the first user behind the scenes. In this way, particularly in a scenario where an instructor makes an advice for a student, communications can be made efficient and active between both parties.
(Aspect 2) According to Aspect 2, in the method of Aspect 1, a first index is associated with the message, for indicating whether the message should be presented to the second user. When the first index indicates that the message should be presented to the second user on the first screen and second screen, the message is placed as a part of messaging by the first user. With the configuration as described, the presentation of the message to the second user can be controlled, thus allowing for more flexible messaging.
(Aspect 3) According to Aspect 3, in the method of Aspect 2, when the first index indicates on the second screen that the message should not be presented to the second user, the message is not placed on the second screen. With the configuration as described, internal interactions can be made between the virtual user and the first user.
(Aspect 4) According to Aspect 4, in the method of Aspect 2 or 3, when the first index indicates that the message should not be presented to the second user, the step of providing the message includes the steps of arranging the message on the first screen together with options including an approval of displaying the message on the second screen; receiving an input from the first user to the options on the first screen; and updating the first index so that the first index indicates that the message should be presented to the second user when the input indicates the approval, whereby the message is placed on the second screen. With the configuration as described, control can be conducted to display only those messages that have got approval from the first user on the second screen of the second user.
(Aspect 5) According to Aspect 5, in the method of Aspect 5, a message displayed on the first screen can be edited by the first user. With the configuration as described, a message intended for the second user can be corrected to be more appropriate.
(Aspect 6) According to Aspect 6, in the method of any of Aspects 1 through 5, a second index is displayed on the first screen and second screen, indicating that the message was provided by the virtual user. With the configuration as described, messages sent to the second user can be distinguished into those generated by the virtual user and those generated by the first user on the displayed screen.
(Aspect 7) According to Aspect 7, the message is in a format including a text and an image.
(Aspect 8) According to Aspect 8, a method is provided for assisting a first user in messaging between the first user and a second user through a talk room. This method includes the steps, executed by a computer, of dynamically generating a message intended for the second user based on the history of messaging in the talk room and health information on the second user; acting on behalf of the first user by providing the message to the talk room in association with the first user; updating the history in response to the provision of the message; and generating a first screen to be presented to the first user and a second screen to be presented to the second user, where the first and second screens are associated with the talk room, wherein the history is classified into a first group involved with the first user having originatead the messaging and the computer, and a second group involved with the second user, and placed on the first screen and second screen, respectively, over time. The virtual user can autonomously determine a next operation in accordance with the progress of the messaging. Also, the virtual user can be clearly demonstrated on a displayed screen, that it is a user for supporting the first user behind the scenes. In this way, the messaging can be promoted in the talk room, particularly, by the virtual user originating the messaging to the second user on behalf of the first user. In a scenario where an instructor make advice for a student, communications can be made efficient and active betwen both parties.
(Aspect 9) According to Aspect 9, in the method of Aspect 8, the step of acting on behalf of the first user includes the steps of arranging the message on the first screen, together with options including approval of displaying the message on the second screen; and receiving an input from the first user for the options on the first screen, wherein when the input indicates the approval, the message is placed on the second screen. With the configuration as described, control can be conducted to display only those messages that have got approval from the first user on the second screen of the second user.
(Aspect 10) According to Aspect 10, in the method of Aspect 9, the message displayed on the first screen can be edited by the first user. With the configuration as described, the message intended for the second user can be corrected to be more appropriate.
(Aspect 11) According to Aspect 12, a program is provided for causing a computer to execute the methtod according to any of Aspects 1 through 10.
(Aspect 12) According to Aspect 12, an information processing device is provided for messaging through a talk room. This information processing device comprises a managemnet unit for managing a relationship between a first user that provides a service and a second user that is provided with a service, and managing a history of messaging between the first user and the second user in a talk room; a virtual user unit for implementing a virtual user for assisting the first user, including an analysis unit for analyzing the history; a message generator unit for dynamically generating a message in accordance with the analysis and health information on the second user; a provision unit for providing the message to the talk room in association with the first user; and an update unit for updating the history with respect to the provided message; and a screen generator unit for generating a first screen to be presented to the first user and a second screen to be presented to the second user, based on the updated history, where the first and second screens are associated with the talk room, wherein the history is classified into a first group involved with the first user originating the messaging and the virtual user, and a second group involved with the second user, and placed on the first and second screens over time. With the configuration as described, the virtual user having a variety of functions can be implemented to behave to support the first user behind the scenes, thus promoting the messaging in the talk room. Also, the virtual user can be clearly demonstrated on a displayed screen, that it is a user for supporting the first user behind the scenes. The virtual user can autonomously determine a next operation in accordance with the progress of the messaging. In this way, particularly in a scenario where a ninstructor makes an advice for a student, communications can be made efficient and active between both parties.

Embodiments of a method, a program, and an information processing device according to the disclosure will hereinafter be described in connection with the accompanying drawings, where the same or similar elements may be designated the same or similar reference numerals, and repetitive descriptions related to the same or similar elements may be omitted in the description of each embodiment. Also, features shown in each embodiment can be applied to other embodiments as long as they are not inconsistent with one anoather.

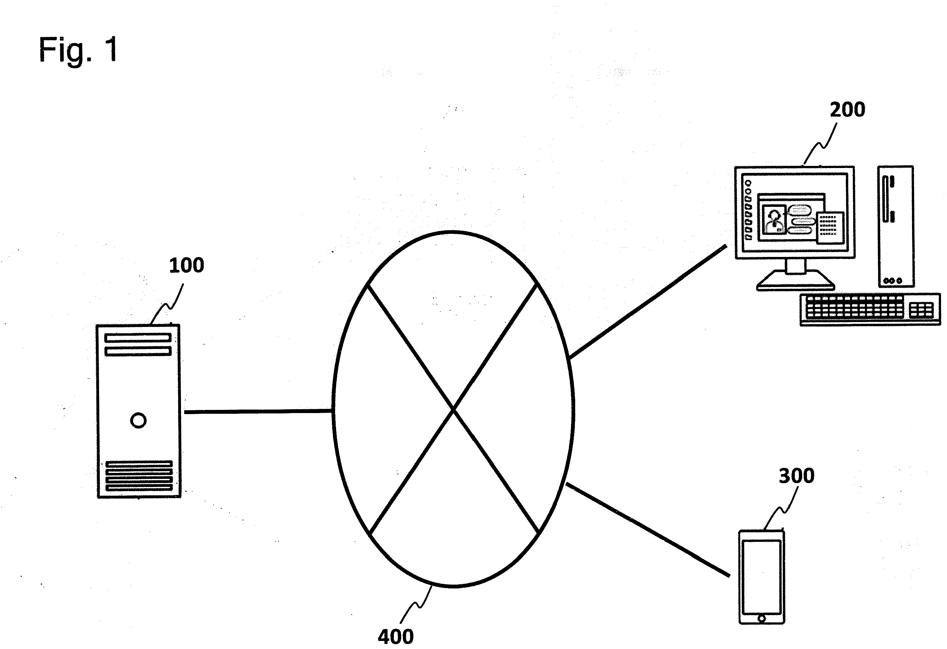

Fig. 1 is a general outline diagram of an information processing system. As shown in Fig. 1, the information processing system comprises a server 100 and a plurality of user terminals 200, 300. The server 100 is connected to the user terminals 200, 300 through a network 400. In Fig. 1, while only two user terminals are illustrated for simplifying the description, the information processing system is not so limited, and may comprise three or more user terminals. Also, specific types of the user terminals 200, 300 are not limited to a personal computer and a smart phone as illustratead, but may also include, for example, electronic devices such as a feature phone, a PDA (Personal Digital Assistant), or a tablet terminal, other than those illustrated.

In the following description on embodiments, assume that a healthcare service is provided on an instant messaging (IM) application. Specifically, in the healthcare service, through messaging (transmission and reception of messages) between a adviser such as a nutrinionist and a healthcare-seeking person, the healthcare-seeking person can receive advice on healthcare from the adviser. In Fig. 1, for example, the user terminal 200 represents a user terminal possessed by the adviser, while the user terminal 300 represents a user terminal possessed by the healthcare-seeking person.

In an exemplary scenario, the healthcare-seeking person transmits, from the user terminal 300, an image of a meal taken by a camera equipped in the user terminal 300 to the healthcare server 100. The server 100 generates a screen for displaying the reaceived image on the user terminal 200. The adviser, when provided with the screen, views the image of the meal of the healthcare-seeking person, displayed on the user terminal 200, and returns a message for the healthcare-seeking person to the user terminal 300 through the server 100.

It should be noted that the application utilized by each user on the user terminals 200, 300 is not limited to the IM application, but may be, for example, SNS (Social Networking Service), Blog or the like. Specifically, according to one embodiment, the user terminals can employ any application that can build a link among accounts of a plurality of users that utilize the healthcare service, and perform messaging (transmission and reception of data such as messages) between the accounts.

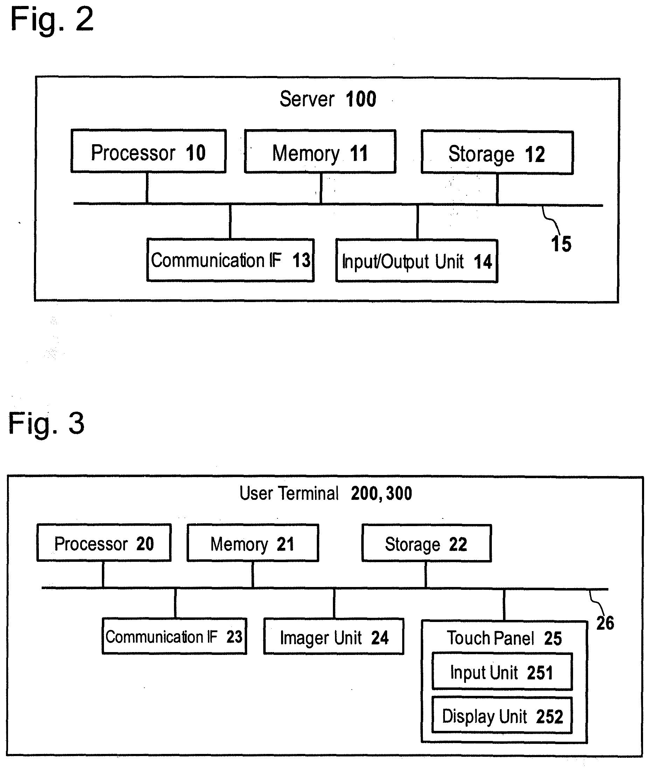

Referring to Figs. 2 and 3, the server 100 and user terminals 200, 300 will be described in regard to their hardware configuration.

Fig. 2 is a hardware configuration diagram of the server 100. The server 100 is an information processing device for providing services through the IM application, and may be, for example, a general-purpose computer such as a workstation and a personal computer, or may be logically implemented by cloud computing. The server 100 comprises a processor 10, a memory 11, a storage 12, a communication interface (IF) 13, input/output unit 14, and the like, all of which are electrically connected to one another through a bus.

The processor 10 is a processing unit which controls the overall operation of the server 100, and also performs information processing and the like required for the control of data transmission/reception between respective elements and for the execution of applications. For example, the processor 10 is a CPU (Central Processing Unit), and executes programs and the like stored in the storage 12 and extended into the memory 11 to implement each information processing.

The memory 11 includes a main memory comprised of volatile storage devices such as DRAM (Dynami Random Access Memory), and an auxiliary memory comprised of non-volatile storage devices such as Flash memory and HDD (Hard Disc Drive). The memory 11 is used as a work area or the like for the processor 10, and stores BIOS (Basic Input/Output System) executed upon start-up of the server 100, a variety of setting information, and the like. The storage 12 stores a variety of programs such as application programs and user authentication program. A database which stores data used for every operation of the IM application may be built in the storage 12.

The communication interface 13 connects the server 100 to the network 400 to communicate with the user terminals 200, 300. The input/output unit 14 represents an information input device such as a mouse and a keyboard, and an output device such as a display. The bus 15 is commonly connected to the respective elements listed above to communicate, for example, address signals, data signals, and a variety of control signals.

Fig. 3 is a hardware configuration diagram of the user terminal 200, 300. The example of Fig 3 is mainly assumed to be a smart phone (300) that has a touch panel. The user terminal 200, 300 comprises a processor 20, a memory 21, a storage 22, a communication interface 23, imager unit 24, a touch panel 25, and the like, all of which are electrically connected to one anoather through a bus 26.

The processor 20 is a processing unit which controls the operation of the user terminal 200, 300, and also performs processing and the like required for the control of data transmission/reception between respective elements and for the execution of applications. For example, the processor 20 is a CPU (Central Processing Unit) and/or a GPU (Graphical Processing Unit) or the like, and implements respective required information processing by executing programs and the like stored in the storage 22 and extended into the memory 21.

The memory 21 includes a main memory comprised of volatile storage devices such as RAM, and an auxiliary storage comprised of non-volatile memory devices such as Flash memory and HDD (Hard Disc Drive). The memory 21 is used as a work area or the like for the processor 20, and also stores BIOS executed upon start-up of the server 100, a variety of setting information, and the like. The storage 22 stores application programs and the like.

The comunication interface 13 connects the user terminals 200, 300 to the network 400 to communicate with the server 100. The communication interface 13 also includes a short-range communication interface such as Bluetooth® and BLE (Bluetooth Low Energy). The imager unit 24, which has a camera function, stores images taken by a user in the storage 22. The touch panel 25 comprises an input unit 251 and a display unit 252. The bus 26 is commonly connected to the respective elements mentioned above, and communicates, for example, address signals, data signals, and a variety of control signals.

The input unit 251 of the touch panel senses an operation on the touch panel 25 by the user. The input unit 251 may employ a pressusre detection type, a resistive-film type, a static capacity type, an electromagnetic induction type, or the like. Then, upon receipt of an input resulting from an operation at an arbitrary location on the touch panel (a physical touch operation to the touch panel such as a touch operation, slide operation, swipe operation, a tap operation, and the like), the touch panel senses a changing amount of pressure, electric resistance, electric capacitance, elastic wave energy, or the like at that location, and identifies the coordiates corresponding to the touched location. The display unit 252 of the touch panel comprises a liquid crystal display or the like.

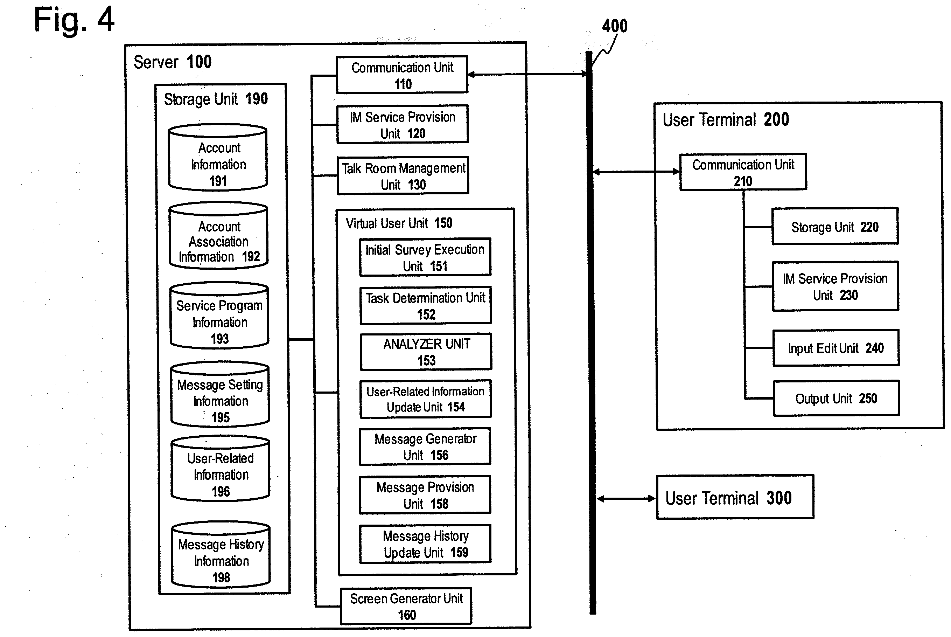

Referring to Fig 4, the information processing system will be described in regard to its functions and configuration. Fig. 4 is an illustrative block diagram showing functions implemented in the server 100 and the user terminal 200, 300, respectively. The server 100 includes a communication unit 110; an IM service provision unit 120; a talk room management unit 130; a virtual user unit 150; a screen generator unit 160; and a storage unit 190, all of which interact with one another. The virtual user unit 150 further includes an initial survey execution unit 151; a task determination unit 152; an analyzer unit 153; a user-related information update unit 154; a message generator unit 156; a message provision unit 158; and a message history update unit 159. The storage unit 190 further manages account information 191, account association information 192, service program information 193, message setting information 195, user-relatead information 196, and message history information 198.

The user terminal 200, 300, on the other hand, includes a communication unit 210; storage unit 220; an IM service provision unit 230; an input editor unit 240; and an output unit 250, all of which interact with one another. The user terminal 300 is similar in function and configuration (hereinafter, user terminals 200, 300 are generally referred to as "user terminal 200" unless otherwise noted).

The communication unit 110 of the server 100 connects the server 100 to the network 400 to transmit/receive data through communications with the user terminal 200. The IM service provision unit 120 provides basic functions for implementing messaging by a plurality of users using the IM application. The talk room management unit 130 manages the account association information 192. Particularly, the talk room management unit 130 manages a relationship between an instructor that provides a healthcare service program and a healthcare-seeking person (student) that is provided with the healthcare service program. The talk room management unit 130 also manages the message history information 198. The virtual user unit 150 implements virtual user functions for assisting the instructor. The screen generator unit 160 generates a user screen associated with a talk room, based on the message history information 198. The user screen is generated for each of the user terminals 200, 300.

The initial survey execution unit 151 of the virtual user unit 150 executes an initial survey when the healthcare-seeking person starts a healthcare service program to acquire initial user-relatead information 196 (particularly, health information) of the healthcare-seeking person. The task determination unit 152 determines a task related to an action of the virtual user, associated with the healthcare service program information 193 and user-related information 196. The analyzer unit 153 analyzes the message history information 198.

The user-ralated information update unit 154 updates the user-relatead information 196 on the healthcare-seeking person and/or instructor in accordance with the analysis on the message history information 198. The message generator unit 156 dynamically generates a message intended for the instructor and/or healthcare-seeking person, associated with the task, in accordance with the analysis on the message history information 198, or based on the updated message setting information 195. The message provision unit 158 provides the generated message to the talk room in association with the instructor. The message history update unit 159 updates the message history information 198 in response to the provision of the message to the talk room.

The storage unit 190 stores a variety of data and programs relatead to the IM application. Specifically, the account information 191 includes user ID's, user names, user types, attribute information, passwords for user authentication, and the like relatead to the instructor and healthcare-seeking person. The account association information 192 includes information related to the association between a plurality of accounts of users that participate in the talk room, for example, the relationship between an instructor that provides a healthcare service program and a healthcare-seeking person that is provided with the healthcare service program. The service program information 193 includes information relatead to the healthcare service program, for example, a service period, task information related to actions that should be performed by the instructor and/or healthcare-seeking person during the period.

The message setting information 195 includes basic information of a message generated by the virtual user in association with a task, for example, a message template, a message category, and a message type, as well as setting information such as a screen display index, and the like. The user-related information 196 includes basic information related to the health of the user involved in the healthcare service program, for example, daily activity information of the healthcare-seeking person, preference information related to hobbies and activities, and the like. In step with the progress of messaging, the user-related information 196 is updated. The message history information 198 includes information related to the history of messaging between a plurality of users that participate in the talk room, for example, a message originator, contents of message, received date and time, and the like.

The communication unit 210 of the user terminal 200 connects the user terminal 200 to the network 400 to transmit/receive data through communications with the server 100. The storage unit 220 stores a variety of data and programs relataed to the IM application, required to utilize the IM service. The IM service provision unit 230 runs the IM application to allow messaging by the user. The input editor unit 240 allows a message to be input and/or editted through the input unit 251 at the time of messaging. The output unit 250 outputs a user screen generatead by the screen generator unit 160 of the server 100 on the display unit 252.

Each component in the respective hardware configuration of the foregoing server 100 and user terminal 200, 300, shown in Figs. 2 and 3, and each functional block shown in Fig. 4 are merely illustrative, and are not limited to those illustrated. Also, each functional block shown in Fig. 4 may be implemented as part of hardware, and/or implemented as part of software. Further, at least part of each functional block 110 - 190 of the server 100 shown in Fig. 4 may be configured to be incorporated in the user terminal 200. On the contrary, at least part of each function 210 - 250 of the user terminal 200 may be configured to be incorporated in the server 100. Stated another way, the method and program according to the embodiment can be executed by a device arbitrarily selected from the server 100 and user terminal 200.

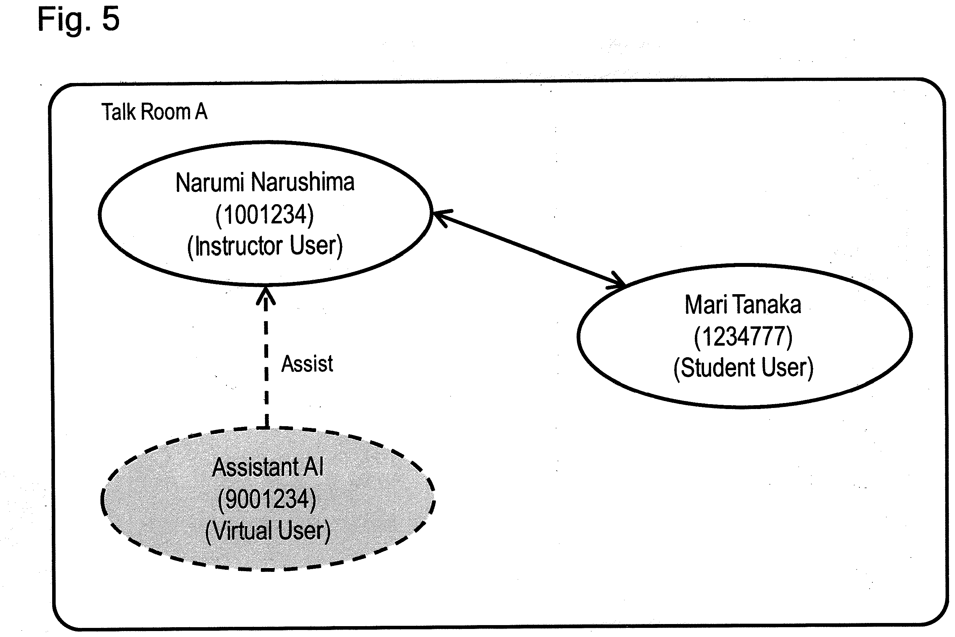

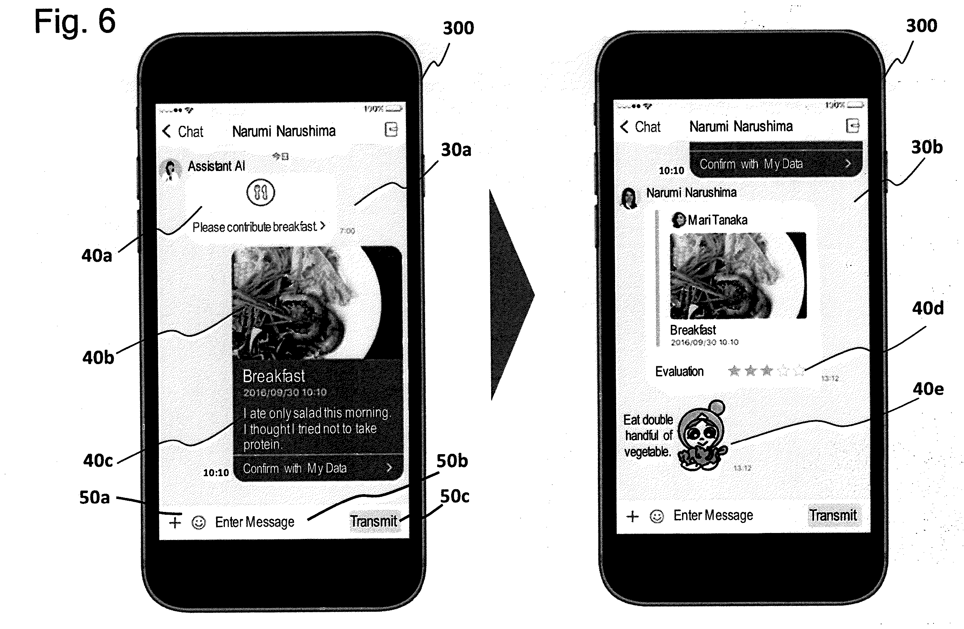

Referring to Figs. 5 and 6, the outline of the healthcare service implemented by the IM application will be described. Fig 5 is an illustrative schematic diagram related to the association of accounts of a plurality of users that have subscribed in the IM application and participated in the talk room. Fig. 6 is an illustrative screen image, related to the healthcare service, where a plurality of users are performing messaging on the IM application.

As shown in Fig. 5, in a talk room A, two users "Narumi Narushima" and "Mari Tanaka" are participating as actual users (users surrounded by solid lines) that operate the user terminal 200. Specifically, "Narumi Narushima" (account ID:1001234) and "Mari Tanaka" (account ID: 1234777) exist as accounts of users that use the IM application. "Narumi Narushima" has a user type "instructor user" (i.e., healthcare instructor), while "Mari Tanaka" has a user type "student user" (i.e., healthcare-seeking person). User basic information such as user name, account ID, and user type may be contained in the account information 192.

Also, a relationship of a healthcare service program provider and a program receiving person exists between "Narumi Narushima" that is an "instructor user" and "Mari Tanaka" that is a "student user." Such a relationship may be contained in the account association information 192. In this regard, two or more "student users" may be associatead with an "instructor user," while two or more "instructor users" may be associated with a single "student user."

In addition to the actual user that operates the user terminal 200, an "assistant UI" (account ID:9001234/user type: virtual user) exists, as implemented by the virtual user unit 150 (user srrounded by a dotted line). The "assistant UI" serves to assist "Narumi Narushima" that is an "instructor user" in the talk room A. As "Narumi Narushima" participates in the talk room A, the "assistant AI" will automatically particilate in the talk room A as well.

The "assistant AI" serves for "Narumi Narushima", for example, by contributing a message for prompting "Mari Tanaka" to join the messaging, and providing an actual message to "Mari Tanaka" on behalf of "Narumi Narushima" in accordance with the progress of the messaging in the talk room A. The "assistant AI" is an agent that autonomously determines a next action in accordance with the progress of the messaging in the talk room A, and takes the action, and is a virtual user equipped with an artificial intelligence (AI) engine. With the existance of the "assistant AI," communications can be made smooth between "Narumi Narushima" and "Mari Tanaka" in the talk room A.

Fig. 6 shows illustrative screens 30a, 30b that are displayed on the user terminal 300 of "Mari Tanaka," associataed with the talk room A. On the screens 30a, 30b, the message history information 198 is classified into a group involved with the opposite party ("Narumi Narushima" and including the "assistant AI" that is a virtual user) that have performed messaging in the talk room A, and a group involved with oneself ("Mari Tanaka"). Then, the message history is placed over time for each of the classified groups. With the progress of messaging, the screen 30a transits to the screen 30b. On the screens 30a, 30b, the group of message history for the opposite party ("Narumi Narushima") is placed along the left side of the screen. Also, the group of message history for oneself ("Mari Tanaka") is placed along the right side of the screen.

Each message included in the message history may be displayed together with an icon image and a user name, as well as a message contributed time. A message provided by the "assistant AI" through the virtual user unit 150 is classified into the same group as "Narumi Narushima" that is her instructor, regarded as provided by "Narumi Narushima". In other words, the message history involved with the "assistant AI" is placed on the left side. A message provided by the "assistant AI" may be placed together with the icon image and user name of the "assistant AI." Alternatively, such a message may be placed together with the icon image and user name of "Narumi Narushima (not the "assistant AI"). Notably, the user terminal 200 of "Narumi Narushima" displays a screen on which the opposite party and herself (i.e., the opposite party ("Mari Tanaka") and herself ("Narumi Narushima")) change places, corresponding to the screens 30a, 30b.

As shown in Fig. 6, each message 40a - 40e is displayed along time series from the top to the bottom of the screen in a ballon form. Each message may be in a text and/or image format, but is not so limited. An audio format or the like may be employed in addition to the text and/or image format. On the screen 30a, the message 40a is dynamically generated by the "assistant AI," and composed of a meal icon and a text including "Let's contribute the breakfast." For example, when the user-related information 196 of "Mari Tanaka" includes information indicating that she takes breakfast at 7:00AM, the "assistant AI" dynamically creates and provides the aforementioned message at 7:00AM. In this way, with the "assistant AI" transmitting a message on behalf of "Narumi Narushima," the messaging can be more efficiently performed in the talk room, and communications can be made more active.

In response to the message 40a, a message 40b is displayed including an image of the breakfast provided by herself ("Mari Tanaka"). Specifically, the message 40b is comprised of the image of the breakfast, an image title, and the time of day ("breakfast" "2016/09/30 10:10"). The time of day may be copied from timestamp information associated with the breakfast image. A message 40c is displayed in conjunction with the message 40b. The message 40c includes a text saying "I ate only salad this morning, because I thaught I tried not to take protein." In this event, the messages 40a and 40b may be transmitted in a single transmission operation by the user such that they are contributed simultaneously.

Turning to the screen 30b, a messasge 40d provided by the opposite party ("Narumi Narushima") is now displayed thereon. The message 40d is comprised of the image of the breakfast provided by herself ("Mari Tanaka") and an evaluation (three stars) for the breakfast, represented by an integer value. In conjunction with the message 40d, a message 40e provided by the opposite party ("Narumi Narushima") is displayed. The message 40d includes a charater image (so-called stamp image).

A message to be transmitted by "Mari Tanaka" is entered from fields provided in a bottom area of the screen. Spefically, the fields include an image input field 50a for selectively inputting a photo or a stamp image; a text input field 50b that allows a text to be input through a software keyboard or the like; and a "transmission" button 50c for transmitting the message.

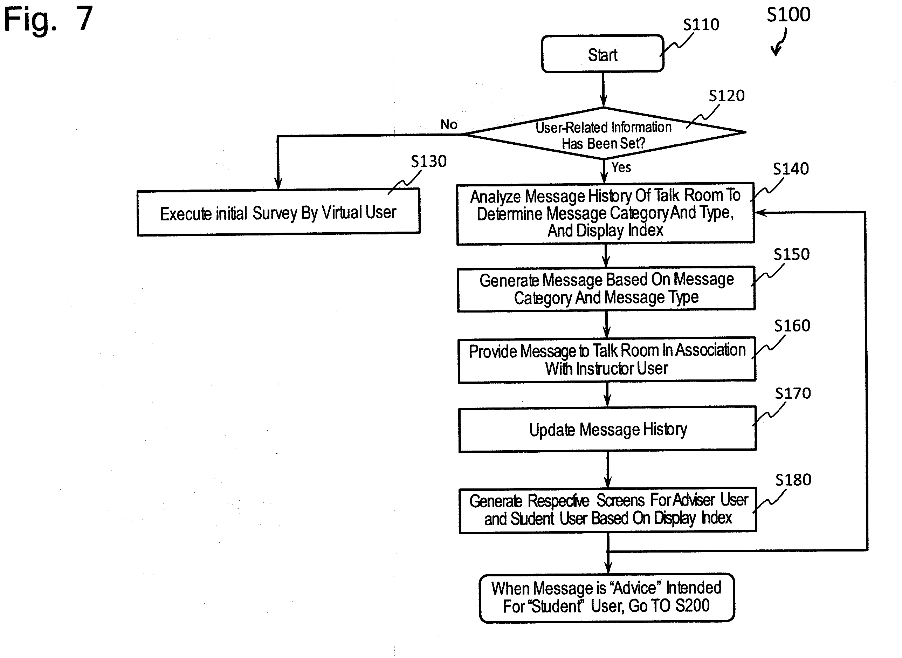

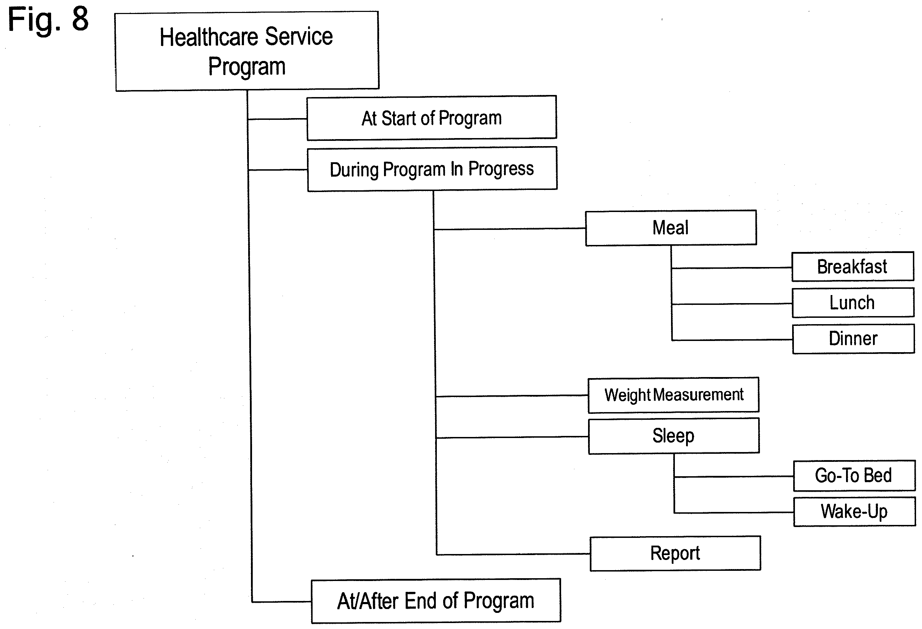

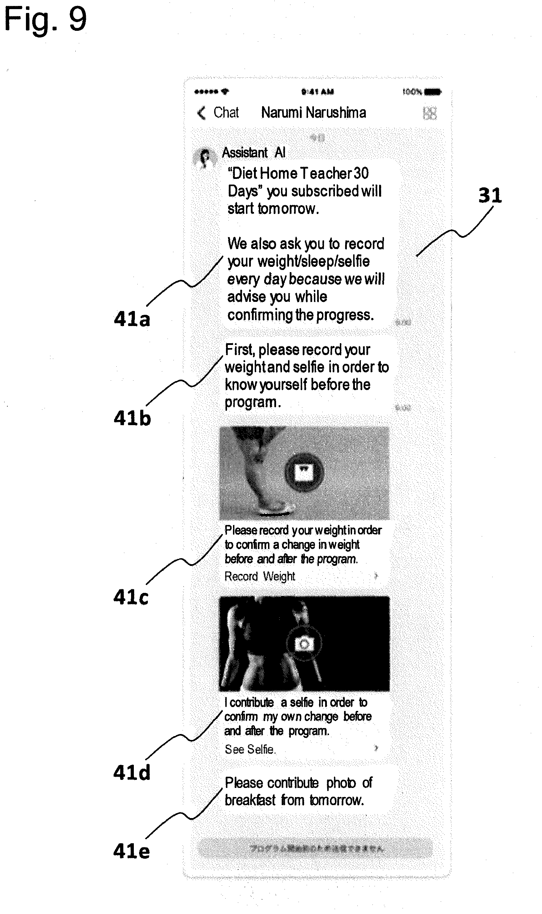

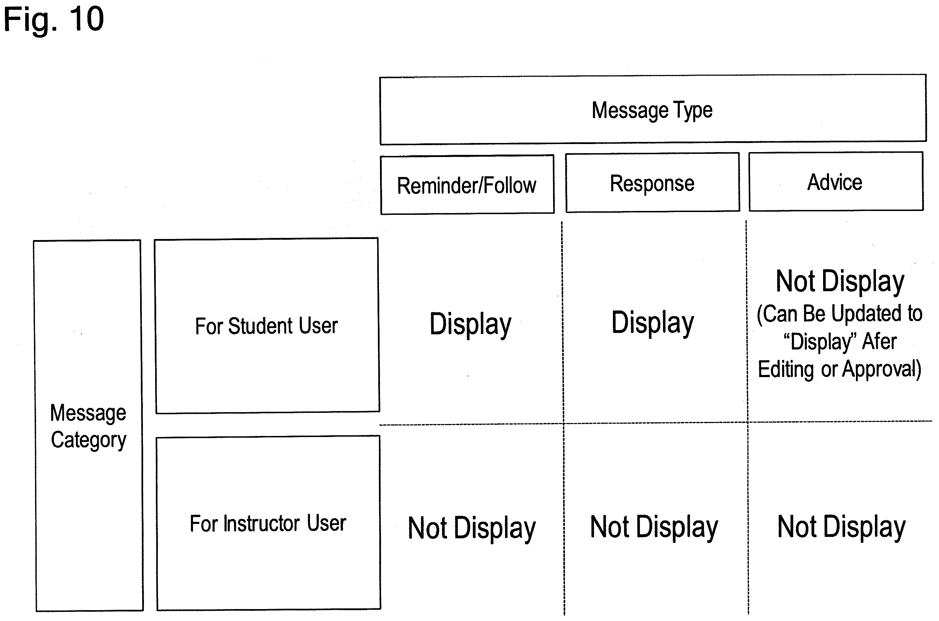

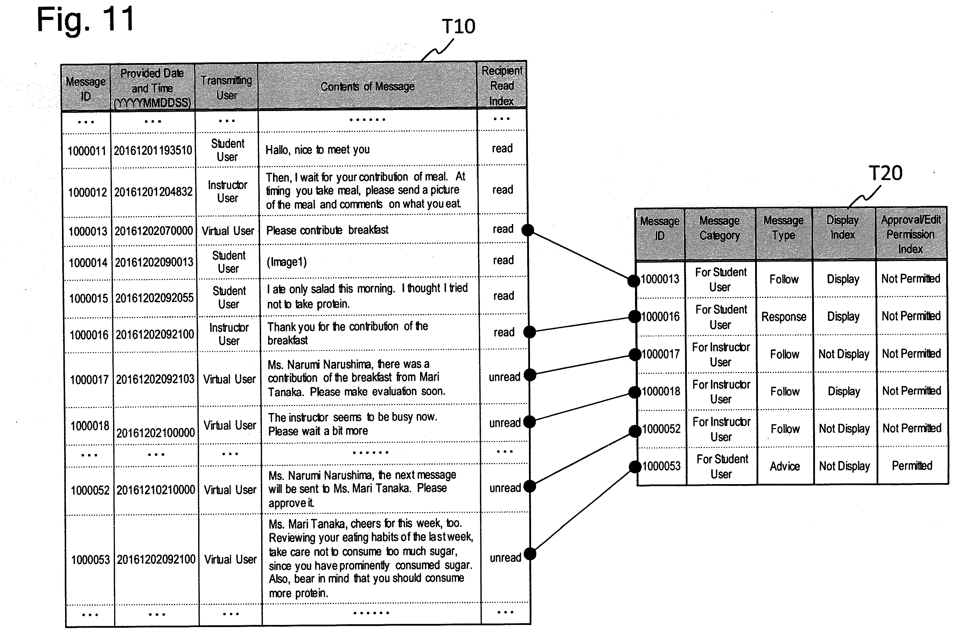

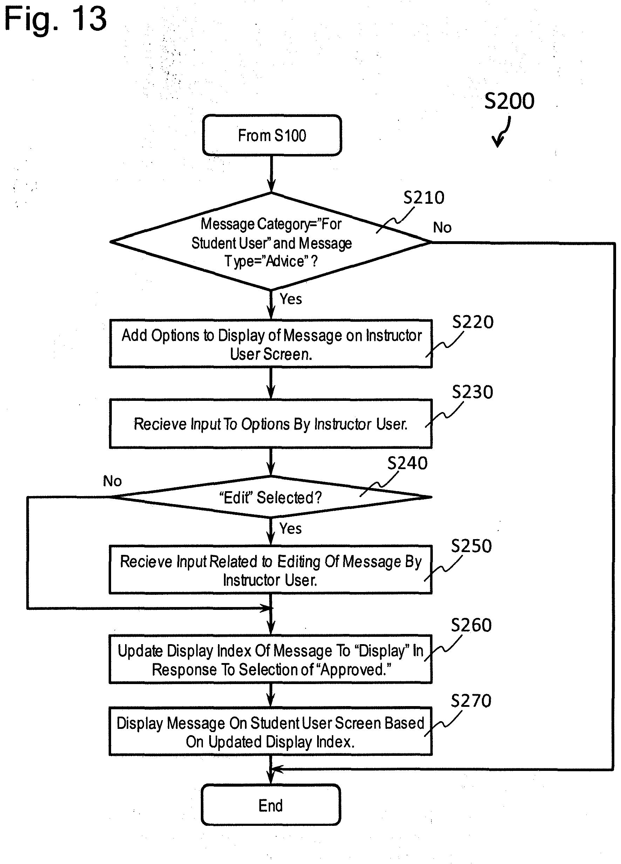

Referring now to Figs. 7 - 14, a method for messaging through a talk room will be described according to one embodiment. Figs. 7 and 13 are illustrative flow diagrams by a virtual user implemented by a method according to one embodiment for assisting an instructor. Fig. 8 is an outline diagram of a task list related to actions which are taken when the virtual user performs the messaging. Figs. 9 - 14 are illustrative screen images generated through the method according to the one embodimente and displayed on the user terminal of the instructor and/or healthcare-seeking person. Fig. 10 is an illustrative schematic diagram relatead to relationshops between the message category, message type, and display index contained in the message setting information 195. Fig 11 is an illustrative tablar configuration diagram related to the message history information 198.

Fig. 7 is a flow diagram showing a basic processing flow S100 for the messaging implemented by the virtual user unit 150. The processing flow S100 is started at S110. For example, the processing flow S100 is started in response to a trigger associated with a task related to an action taken by the virtual user for messaging, in association with the healthcare service program shown in Fig. 5 and 6. The task is defined in the service program information 193 illustrated in Fig. 8, and determined by the task determination unit 152. An exemplary task is defined for each of "at the start of the program," "during the program in progress," and "at/after the end of the program." Also, a task "during the program in progress" is defined as "meal," "measurement of weight," "sleep," and "report." Further, the "meal" is defined as "breakfast," "lunch," and "dinner," the "sleep" is defined as "go to bed" and "wake up," and each task is associated with a variety of triggers (for example, a predetermined time, period, user operation, and the like).

As the processing flow S100 is started at S110, the processing branches depending on whether or not user-related information has been set for a student user (S120). If the user-relatead information has not yet been set (No at S120), the task is "at the start of the program," so that an initial survay is made by the initial survey execution unit 151 (S130). In this way, initial user-related information is acquired.

If the user-related information has already been set (Yes at S120), the task is "during the program in progress" or "at/after the end of program," and the processing flow proceeds to processing of messaging (S140 - S180). Specifically, the analyzer unit 153 analyzes the message history of the talk room to determine the message category, message type, and display index (S140). The message generator unit 156 dynamically generates a message associated with the task based on the message category and message type that have been determined based on the message history information 198 (S150). The message provision unit 158 provides the generated message to the talk room in association with the instructor user (S160). The message history update unit 159 updates the message history information 198 in response to the provision of the message (S170). Then, the screen generator unit 160 generates a screen to be presented to the instructor user, and a screen to be presented to the student user, associated with the talk room (S180).

In the initial survey at S130, the initial survey execution unit 151 provides the talk room with a question message that has been previously determined in the task "at the start of program." The initial survey is proceeded by the student user providing a reply message to the question message. In this way, initial user-related information 196 is automatically acquired and set for the student user. Fig. 9 is a screen showing an exemplary progress of the initial survey in the talk room A. Here, similar to Fig. 5, assume that the instructor user "Narumi Narushima" and the student user "Mari Tanaka" participate in the talk room A, where "Mari Tanaka" has subscribed in a service program for a "Diet Home Teacher 30 days" course. The initial survey is performed at a day at which the service program is started.

On a screen 31 of "Mari Tanaka" associated with the talk room A of Fig. 9, messages 41a - 41e, provided by the "assistant AI" on behalf of "Narumi Narushima," are displayed. The messages 41a - 41e are generated based on information contained in the service program information 193. The messages 41a and 41b display an announcement of the start of the service program for the "Diet Home Teacher 30 Days" course, and a message for prompting the student user "Mari Tanaka" to register her user-rested information. The user-related information 196 includes, for example, in regard to the student user "Mari Tanaka," the weight, life style such as meal times and frequency of exercise, preferences, selfie (her own image), and the like.

The message 41c prompts the student user "Mari Tanaka" to enter her weight, and the message 41d prompts her to enter her selfie. In response, the student user "Mari Tanaka" enters her weight and selfie, resulting in registration of the initial user-related information 196. In this regard, the user-related information 196 may be personalized with the progress of the service program. Stated another way, the user-related information update unit 154 updates the user-related information 196 at all times in accordance with the history of messages subsequently provided from the student user "Mari Tanaka." The message 41e displays an announcement related to subsequent tasks contained in the service program information 193. The announcement displayed herein relates to a task "breakfast." The message 41a - 41e by the virtual user, and the reply message by "Mari Tanaka" are registered in the message history information 198. In this way, the virtual user performs the initial survey on behalf of the instructor user, thus allowing for a higher efficiency of communications in the talk room.

At S140, the analyzer unit 153 analyzes the message history information 198 to determine the message category, message type, and display index contained in the message setting information 195, for a message that is to be transmitted in accordance with a task. The analysis is made by widely applying information processing technologies related to artificial intelligence such as parsing, natural language processing, image parsing, and the like on the message history information 198. Fig. 10 shows an illustrative relationship between the message category, message type, and display index. As shown in Fig. 10, the message category includes "For Student User" and "For Instructor User," and determines which user a message is intended for. The message type includes "Reminder/Follow," "Response," and "Advice." For example, in "Reminder/Follow," the virtual user prompts a student user at a predetermined time with respect to a task, or follows the student user when a predetermined period has elapsed since a previous contribution. In "Response," a student user or an instructor user immediately responses to a provided message. In "Advice," a student user or an instructor use is advised at a predetermined time or at predetermined intervals. For a message generated by the virtual user, its pattern is determined in combination of the message category and message type. In this example, there are six patterns.

The pattern of the message is further set in association with the display index. The display index is an index for indicating whether a generated message should be presented to a student user, i.e., whether the message should be displayed on the screen of the student user, and includes "Display" or "Not Display." Any message may be presented to an instructor user at all times irrespective of the display index. Giving, as an example, the message 40a shown in Fig. 6 which has the message category set to "For Student User" and the message type set to "Reminder/Follow," its display index is set to "Display," so that the message 40a is displayed on the screen of the student user. On the other hand, a message having the message category set to "For Student User" and the message type set to "Advice" is not displayed on the screen of the student user until the message is edited or approved by the instructor user, and the initial display index is set to "Not Display." In this event, the display index is updated to "Display" when the message is edited or approved by the instructor user (later described). It should be noted that the message category, message type, and display index as described are not limited to the foregoing. For example, the message type "Reminder/Follow" may be set separately from "reminder" and "follow."

At S150, the message generator unit 156 generates a message using a message template pursuant to a task, contained in the message setting informationm 195, based on the message category and message type. For example, the aforementioned message 40a relates to the "breakfast" task, and as a message having the message category set to "For Student User" and the message type set to "Reminder/Follow," the message template "Let's contribute the breakfast" is applied as it is to the message. The message template may be customized and/or personalized based on items contained in the account information 191 and/or user-relatead information 196.

At S160, the message provision unit 158 provides the generated message to the talk room at a predetermined timing in association with the instructor user. For example, when the message has the message category set to "For Student User," the message is provided as that from an instructor user, like the message 40a saying "Let's contribute the breakfast." Also, when the message has the message type set to "reminder" like the aforementioned message 40a, the timing at which the message is provided is determined based on the user-relatead information 196. For example, the timing may be dynamically determined based on the time of the breakfast acquired from the student user through the initial survey, or a time personalized by the actual time of the breakfast later than the acquired time.

At S170, the message history update unit 159 adds the history of the message provided at S160 to the message history information 198. Fig. 11 shows an illustrative message history table T10 and virtual user message history table T20. The message history information 198 is comprised of respective data for each record. The message history table T10 contains, in its items, message ID, message provision date and time, transmitting user (student user, instructor user, or virtual user), contents of message (contents of text or image format), recipient read index (read or unread), and the like. Also, the virtual user message history table T20 contains, in its items, message ID, message category (For Student User or for Instructor User), message type (reminder, follow, response, advice), display index (display or not display), approval/edit permission index (Yes or No), and the like. The message history table T10 and virtual user message history table T20 are associated with each other with the message ID used as a key.

At S180, the screen generator unit 160 generates an instructor user screen to be presented to the instructor user and a student user screen to be presented to the student user, respectively, using the message history information 198 updated at S170, where the screens are associated with the talk room. On each of the instructor user screen and student user screen, contents of messages in the message history information 198 are classified and placed over time for each of the instructor user and student user that have performed the messaging. Also, as to messages provided by the virtual user, when such messages have the display index set to "Display" on the virtual user message history tabel T20 (i.e., messages presented to the student user), these messages are classified into the same group as the instructor user, as messaging by the instructor user, and their message history is placed in this group. When a message has the display index set to "Not Display," the message is not placed on the student user screen. Stated another way, the message is displayed on the instructor user screen, in such a manner that the virtual user provides the message only to the instructor user. After S180, when the message has the message category set to "For Student User" and the message type set to "Advice," the processing flow S100 subsequently continues to a processing flow S200 in Fig. 13. Otherwise, the processing at S140 - S180 are repeated in sequence.

As can be seen in the examples of the screens 30a, 30b shown in Fig. 6, on the student user screen, massages by the "assistant AI" and "Narumi Narushima" are mixed on the left column which presents messaging by the instructor user. In this way, an index (for example, an icon and the user) may be displayed for indicating that a message was provided by the "assistant AI," or as an alternative, the icon and user ID may be unified as messages by "Narumi Narushima."

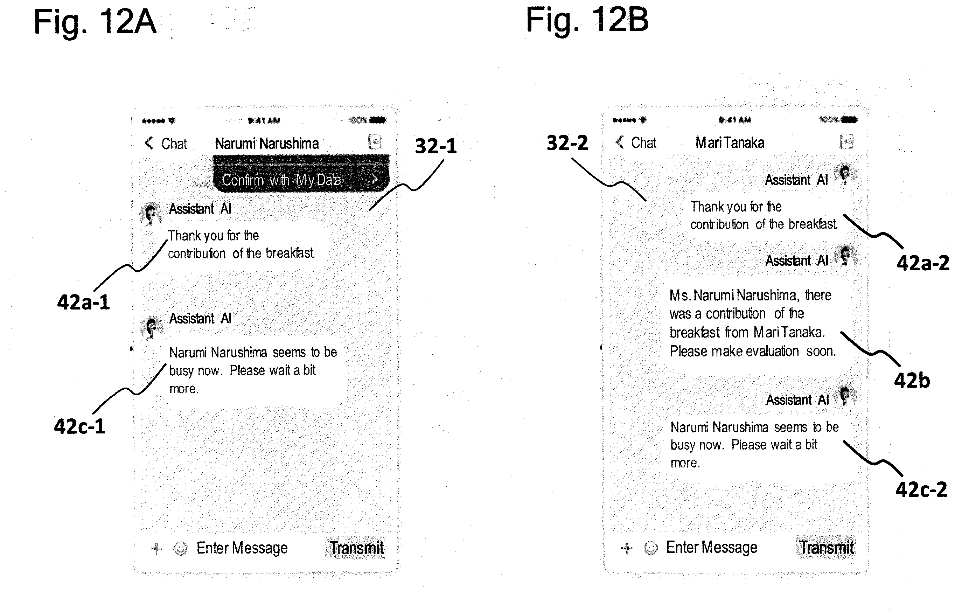

Referring to Figs. 12A and 12B, illustrative screen images will be described as generated when a sequence of information processing S140 - S180, included in the processing flow S100 of Fig. 7, were executed in repetition. Fig. 12A shows a student user screen 32-1 in the talk room A, while Fig. 12B shows an instructor user screen 32-2 corresponding thereto. Assume herein a scenario where in the "breakfast" task, when the student user "Mari Tanaka" has provided a breakfast image message to the talk room A, the virtual user "assistant AI" supports the instructor user "Narumi Narushima" until the instructor user "Narumi Narushima" evaluates the breakfast.

First, the "assistant AI" analyzes the history of the breakfast image message by "Mari Tanaka." Then, as the next messaging related to the "breakfast" task, the "assistant AI" determines that it should generate a message, the message category of which is "For Student User," and the message type of which is "Response" (S140). Then, based on such information, the "assistant AI" dynamically generates a message saying "Thank you for the contribution of the breakfast!" (S150), and provides the same to the talk room A (S160). The "assistant AI" also updates the message history (S170). Since the message has its display index set to "Display" (Fig. 10), the messages 42a-1 and 42a-2 are displayed on the student user screen 32-1 of "Mari Tanaka" and the adviser user screen 32-2 of Narumi Narushima" (S180).

Returning to S140, the "assistant AI" then analyzes the message history of the aforementioned "Thank you for the contribution of the breakfast!," and determines that "Narumi Narushima" should be rprompted. Specifically, the "assistant AI" determines that the messaging is required to have the message category set to "For Instructor User" and the message type set to "reminder" (S140). Next, based on such information, the "assistant AI" dynamically creates a message saying "Ms. Narumi Narushima, the breakfast was contributed from Ms. Mari Tanaka. Please evaluate it soon" (S150), provides this messaage to the talk room A (S160), and then updates the message history (S170). Since this message has the display index set to "Not Display" (Fig. 10), the message 42b is not displayed on the student user screen 32-1 of "Mari Tanaka," but is only displayed on the instructor user screen 32-2 of "Narumi Narushima" (S180).

Returning again to S140, the "assistant AI" then analyzes the message history of the aforementioned "Ms. Narumi Narushima, the breakfast was contributed from Ms. Mari Tanaka. Please evaluate it soon." Then, if the instructor user "Narumi Narushima" has not yet carried out the evaluation even after the lapse of a predetermined time, the "assistant AI" determines that it should support "Mari Tanaka." Specifically, the "assistant AI" determines that the messaging is rrequired to have the message category set to "For Student User" and the message type set to "follow" (S140). Then, based on such information, the "assistant AI" dynamically creates a message saying "Narumi Narushima seems to be busy now. Pleast wait a bit more" (S150), and provides this message to the talk room A (S160). The "assistant AI" also updates the message history (S170). Since this message has the display index set to "Display" (Fig. 10), the messages 42c-1 and 42c-2 are displayed on the student user screen of "Mari Tanaka" and the instructor user screen of "Narumi Narushima" (S180).

As will be understood by referring to Figs. 12A and 12B, while the student user screen 32-1 and instructor user screen 32-2 correspond to each other, messages displayed on the respective screens may sometimes not correspond depending on the control by the display index. Specifically, the message 42b shown in Fig. 12B is displayed on the instructor user screen 32-2, but not displayed on the student user screen 32-1. In this way, the virtual user not only creates messages intended for the student user on behalf of the instructor user, but also provides messages displayed only for the instructor user, thus making it possible to promote the messaging in the talk room and make communications more active.

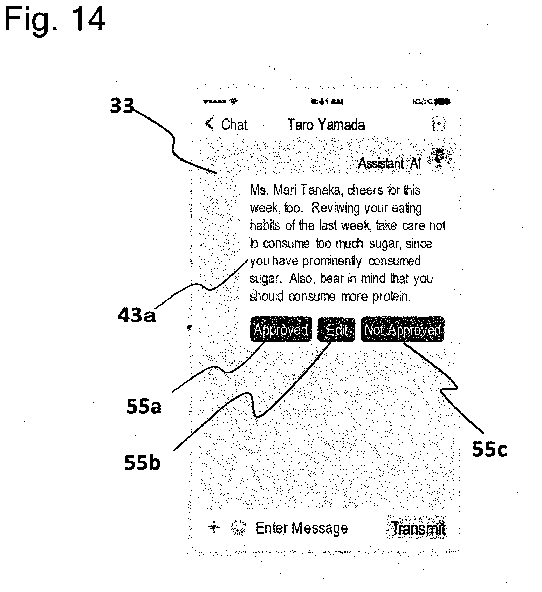

As an implementation for providing a message displayed only for the instructor user, a processing flow S200 of Fig 13 will now be described in connection with a screen 33 of Fig. 14. The processing flow 200 of Fig. 13 is taken over from the processing flow S100 of Fig. 7. Specifically, assume that an example of the message 43a generated at S150 of Fig. 7 says "Ms. Mari Tanaka, cheers for this week, too! Reviwing your eating habits of the last week, you have prominently consumed sugar, so take care not to consume too much sugar. Also, bear in mind that you should consume more protein." The message 43a is generated based on the history information on past messaging between the instructor user and the student user and also based on updated health information on the student user. Also, the message 43a has the message category set to "For Student User" and the message type set to "Advice," and its display index is set to "Not Display" (Fig. 10). Stated another way, at S180, the message 43a is not displayed on the student user screen, but is displayed only on the adavisor user scren 33 (Fig. 14).

In the processing flow S200 of Fig. 13, the message provision unit 158 first determines whether a message of interest has the message category set to "For Student User" and the message type set to "Advice" (S210). If Yes at S210, the screen generator unit 160 displays the message with options further added thereto on the instructor user screen (S220). In the example of Fig. 14, on the instructor user screen 33, respective buttons "Approved" 55a, "Edit" 55b, and "Not Approved" 55c are added as options in addition to the message 43a. The instructor user can select one from these buttons 55a - 55c through an operation on the touch panel.

Next, on the instructor user screen, the input unit 251 receives an input as to the options (S230). If the input selects "Edit" (S240), the message displayed on the instructor user screen can be edited by the instructor user. Specifically, the input unit 251 receives an input for editing the message (S250). In the example of Fig. 14, by selecting "Edit" 55b, the instructor user can freely modify the message 43a generated by the virtual user to be more appropriate one in accordance with actual stuation of the student user.

After the message is edited at S250, the input unit 251 further receives an input related to approval of the message from the instructor user. If No at S240, the editing of the message at S250 may be bypassed, and the input unit 251 may immediately receive the input related to the approval of the message. In response to the reception of the input approval, the message provision unit 158 updates the display index of the message of interest from "Not Display" to "Display" (S260). The screen generator unit 160 newly places the message of interest on the student user screen in accordance with the updated display index. Stated another way, the message of interest is displayed on the student user screen (S270).

In this event, if the instructor user selects "Not Approved" 55c, the display index of the message of interest will be kept being "Not Display," so that the message of interest will not be placed on the student user screen. In this way, in the implementation of Figs. 13 and 14, when a message has the message category set to "For Student User" and the message type set to "Advice," this message is first displayed on the instructor user screen, and then displayed on the student user screen only after the message is editted and/or approved by the instructor user. In this way, more appropriate messaging can be accomplished for the student user.

According to the embodiment described above, a virtual user having a variety of functions can be implemented for assisting an instructor user behind the scenes. The virtual user can autonomously determine a next operation in accordance with the progress of messaging. In this way, the messaging can be promoted in a talk room particularly by the virtual user performing the messaging to the student user on behalf of the instructor user. Communications can be made efficient and active between the instructor user and the student user. Also, a message generated by the virtual user is made editable, thereby allowing the messaging to be more appropriate.

The embodiments described above are merely illustrative for facilitating the understanding of the invention, and is not intended to interpret the invention in a limiting sense. It should be understood that the present invention can be modified or altered without departing from its scope and spirit, and encompasses equivalents thereof.

## Claims

1. A method for messaging through a talk room, comprising the steps, performed by a virtual user implemented by a computer for assisting a first user, of:
dynamically generating a message intended for the first user or the second user based on a history of messaging in the talk room and health information on a second user;
providing the message to the talk room in association with the first user;
updating the history in response to the provision of the message; and
generating a first screen to be presented to the first user and a second screen to be presented to the second user, said first and second screens being associated with the talk room,
wherein the history is classified into a group involved with the first user and the virtual user that perform the messaging, and a group involved with the second user, and placed on each of the first screen and the second screen over time.

2. A method according to claim 1, wherein:
said first index as to whether said message should be presented to the second user is associated with said message, and
on said first screen and said second screen, when said first index indicates that said message should be presented to the second user, said message is placed as a part of messaging by the first user.

3. A method according to claim 2, wherein:
when said first index indicates that said message should not be presented to the second user, said message is not placed on said second screen.

4. A method according to claim 2 or 3, wherein:
when said first index indicates that said message should not be presented to the second user, the step of providing said message includes the steps of:
displaying said message on said first screen together with options including an approval of displaying said message on said second screen;
receiving an input from the first user to the options on said first screen; and
when said input indicates the approval, updating said first index such that said first index indicates that said message should be presented to the second user,
wherein said message is placed on said second screen.

5. A method according to claim 4, wherein said message displayed on said first screen can be edited by the first user.

6. A method according to any of claims 1 through 5, wherein:
on said first screen and said second screen, a second index is displayed to indicate that said message was provided by said virtual user.

7. A method according to any of claims 1 through 6, wherein said message is in a format including a text and an image.

8. A method for assisting a first user in messaging between the first user and a second user through a talk room, said method comprising the steps, performed by a computer, of:
dynamically generating a message intended for the second user based on a history of messaging in the talk room and health information on the second user;
acting on behalf of the first user by providing said message to the talk room in association with the first user;
updating the history in response to the provision of said message; and
generating a first screen to be presented to the first user and a second screen to be presented to the second user, said first and second screens being associated with the talk room,
wherein said history is classified into a group involved with the first user and said computer that perform the messaging, and a group involved with the second user, and placed on each of said first screen and said second screen over time.

9. A method according to claim 8, wherein said step of acting on behalf of the first user includes the steps of:
displaying said message on said first screen together with options including an approval of displaying said message on said second screen; and
receiving an input from the first user to said options on said first screen,
wherein when said input indicates the approval, said message is placed on said second screen.

10. A method according to claim 9, wherein said message displayed on said first screen can be edited by the first user.

11. A program for causing said computer to perform the method according to any of claims 1 through 10.

12. An information processing device for messaging through a talk room, comprising:
a management unit for managing a relationship between a first user that provides a service and a second user that is provided with the service, and managing a history of messaging between the first user and the second user in said talk room;
a virtual user unit for implementing a virtual user for assisting the first user, including;
an analyzer unit for analyzing the history;
a message generator unit for dynamically generating a message in accordance with the analysis and health information on the second user;
a provision unit for providing the message to said talk room in association with the first user; and
an update unit for updating the history with respect to the provided message; and
a screen generator unit for generating a first screen to be presented to the first user and a second screen to be presented to the second user based on the updated history, said first and second screens being associated with the talk room,
wherein said history is classified into a group involved with the first user and said virtual user that perform the messaging, and a group involved with the second user, and placed on said first screen and said second screen over time.
